(19) Europäisches Patentamt
European Patent Office
Office européen des brevets

(11) Veröffentlichungsnummer: **0 003 975**
**B1**

(12) # EUROPÄISCHE PATENTSCHRIFT

(45) Veröffentlichungstag der Patentschrift: **13.05.81**

(21) Anmeldenummer: **79100426.0**

(22) Anmeldetag: **13.02.79**

(51) Int. Cl.³: **C 07 C 103/58,** C 07 C 103/60, C 07 C 103/64, A 01 N 37/18 // C07D307/52, C07D307/04, C07D309/20

(54) **Dichlormaleinsäurediamid-Derivate, Verfahren zu ihrer Herstellung und ihre Verwendung als Fungizide.**

(30) Priorität: **23.02.78 DE 2807662**

(43) Veröffentlichungstag der Anmeldung: **19.09.79 Patentblatt 79/19**

(45) Bekanntmachung des Hinweises auf die Patenterteilung: **13.05.81 Patentblatt 81/19**

(84) Benannte Vertragsstaaten: **BE CH DE FR GB IT NL SE**

(56) Entgegenhaltungen:
**DE-A-1 806 291**
**DE-A-2 156 967**
**DE-A-2 451 512**
**US-A-2 663 665**
**JOURNAL FÜR PRAKTISCHE CHEMIE, Band 130, 1931 Leipzig L. LEDER «Über Chloride und andere Derivate der Dichlormaleinsäure» Seiten 255 bis 287**

(73) Patentinhaber: **BAYER AG, Zentralbereich Patente, Marken und Lizenzen, D-5090 Leverkusen 1, Bayerwerk (DE)**

(72) Erfinder: **Bonse, Gerhard, DR., Wolfskaul 3, D-5000 Köln 80 (DE)**
Erfinder: **Blank, Heinz Ulrich, Dr., Am Geusfelde 35, D-5068 Odenthal (DE)**
Erfinder: **Brandes, Wilhelm, Dr., Eichendorffstrasse 3, D-5653 Leichlingen 1 (DE)**
Erfinder: **Paul, Volker, Dr., Ahornstrasse 5, D-5650 Solingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



ACTORUM AG.

# Dichlormaleinsäurediamid-Derivate, Verfahren zu ihrer Herstellung und ihre Verwendung als Fungizide

Die vorliegende Erfindung betrifft neue Dichlormaleinsäurediamid-Derivate, Verfahren zu ihrer Herstellung sowie ihre Verwendung als Fungizide.

Es ist bereits bekannt geworden, dass das N-(p-Fluorphenyl)-2,3-dichlormaleinimid eine sehr gute Wirkung gegen Citrusschorf besitzt (vgl. z.B. DT-OS 2156967 bzw. US-PS 3734927). Die Wirkungsbreite der genannten Verbindung ist jedoch bei niedrigen Aufwandmengen nicht voll befriedigend. Weiterhin ist als Standardpräparat mit fungizider Wirkung das Zink-äthylen-1,2-bis-dithiocarbamidat schon seit geraumer Zeit allgemein bekannt (R. Wegler, «Chemie der Pflanzenschutz- und Schädlingsbekämpfungsmittel», Springer-Verlag, Berlin/Heidelberg/New York (1970), Band 2, Seite 65).

Es ist weiterhin schon seit längerer Zeit bekannt, dass bei der Acylierung von Anilin mit Dichlormaleinsäuredichlorid ein Produktgemisch entsteht (vgl. hierzu J. prakt. Chemie 130, 255 (1931)). Wenn auch – wie seit neuerer Zeit zusätzlich bekannt ist – dass Dichlormaleinsäuredichlorid ausschliesslich in der lactoiden (cyclischen) Form als Tetrachlor-5H-furan-2-on vorliegt, so können Umsetzungen lactoider Säurechloride von Dicarbonsäuren mit nucleophilen Reaktionspartnern prinzipiell zu Produkten führen, die einen intakten lactoiden Ring enthalten (Chem. Ber. 104, 3378 (1971)) oder unter Ringöffnung offenkettige Derivate ergeben (Chem. Ber. 109, 1163 (1976)). Dementsprechend wird in der Literatur (J. prakt. Chem. 130, 25 (1931)) bei der Acylierung von Anilin mit Dichlormaleinsäuredichlorid jeweils ein Produktgemisch beschrieben, und zwar ein gelbes Produkt mit Fp. 170°C und ein weisses Produkt mit Fp. 193°C, beide Produkte besitzen die gleiche Bruttoformel $C_{16}H_{12}Cl_2N_2O_2$. Die alleinige Bildung von weissem Produkt (Fp. 193°C), welches als symmetrisches Dichlormaleinsäure-bis-phenylamid beschrieben wird, war nur dann möglich, wenn das Dichlormaleinsäuredichlorid zuvor einer Behandlung mit Aluminiumchlorid unterworfen wurde, was als Umwandlung der lactoiden in die offenkettige symmetrische Form gedeutet wurde.

Von dem oben erwähnten, vorbeschriebenen Dichlormaleinsäure-bis-phenylamid (J. prakt. Chem. 130, 255 (1931)) sind bisher keine biologischen Wirkungen bekannt geworden.

Es wurden nun als neue Verbindungen die Dichlormaleinsäurediamid-Derivate mit der allgemeinen Formel

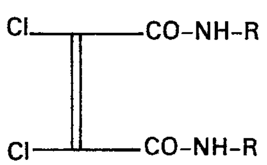

(I)

in welcher

R für einen gegebenenfalls substituierten aliphatischen Rest mit bis zu 8 Kohlenstoffatomen, für einen Cycloalkylrest mit 3 bis 6 Kohlenstoffatomen oder für einen substituierten Phenylrest steht,

gefunden. Sie weisen starke fungizide Eigenschaften auf.

Weiterhin wurde gefunden, dass man die Dichlormaleinsäurediamid-Derivate der Formel (I) erhält, wenn man Halogenide der Dichlormaleinsäure mit der Formel

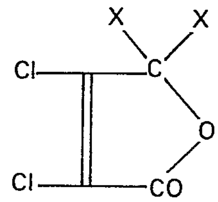

bzw.

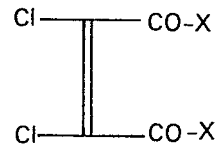

(IIa) (IIb)

in welcher

X für Halogen steht,

mit einem Amin der allgemeinen Formel

$H_2N-R$ (III)

in welcher

R die oben angegebene Bedeutung besitzt,

in einem Verdünnungsmittel in Gegenwart eines Säurebindemittels umsetzt.

Wie schliesslich noch gefunden wurde, lässt sich ein einheitlich zu den offenkettigen symmetrischen Verbindungen der allgemeinen Formel (I) führender Reaktionsablauf (auch bei Ausgang von Verbindungen des lactoiden Typs IIa) leicht dadurch bewerkstelligen, dass man das zu acylierende Amin der Formel (III) im Reaktionsgefäss vorlegt und das Dichlormaleinsäurehalogenid (Formel IIa bzw. IIb) anschliessend zugibt.

Überraschenderweise zeigen die erfindungsgemässen Dichlormaleinsäurediamid-Derivate der Formel (I) eine erheblich höhere fungizide Wirkung als die aus dem Stande der Technik bekannten Verbindungen. Die erfindungsgemässen Stoffe stellen somit eine Bereicherung der Technik dar.

Weiterhin ist überraschend, dass durch die erfindungsgemässe Reihenfolge der Zugabe in einfacher Weise erreicht wird, dass einheitliche Produkte der Formel (I) entstehen. Der Wegfall der nach den bisherigen Kenntnissen notwendigen Behandlung des Dichlormaleinsäurehalogenids mit Aluminiumchlorid oder einer anderen Lewis-Säure bedeutet eine technische Vereinfachung.

Verwendet man Tetrachlor-5H-furan-2-on («Dichlormaleinsäuredichlorid») und Äthylamin als Ausgangsstoffe, so lässt sich der Reaktionsablauf durch das folgende Formelschema wiedergeben:

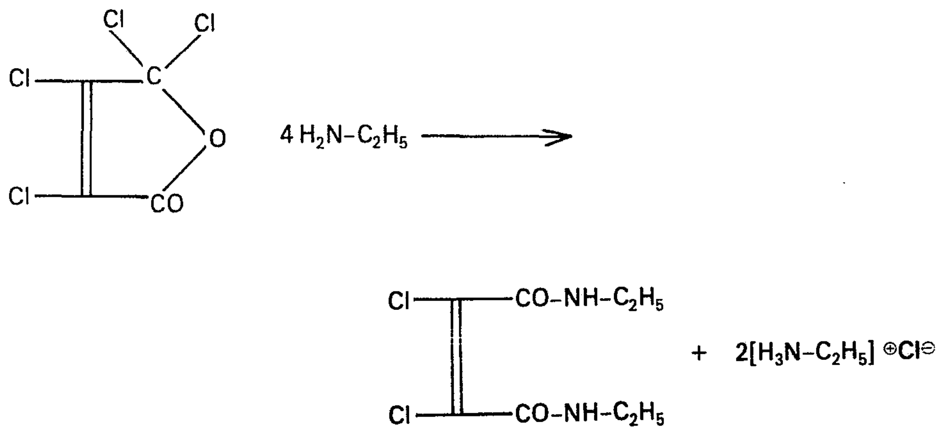

Die als Ausgangsstoffe zu verwendenden Halogenide der Dichlormaleinsäure sind durch die Formel (II) angegeben. In dieser Formel steht X vorzugsweise für Chlor. Die Verbindungen sind bekannt und lassen sich nach allgemein bekannten Verfahren herstellen, so z.B. durch Halogenierung von Dichlormaleinsäureanhydrid, z.B. unter Verwendung von Phosphorpentachlorid, Phosgen und Thionylchlorid, oder z.B. durch Verwendung von Halogen, wie Chlor, in Gegenwart von Katalysatoren, wie z.B. Eisensalzen.

Die weiterhin als Ausgangsstoffe benötigten Amine sind durch die Formel (III) allgemein definiert. In dieser Formel steht R vorzugsweise für einen geradkettigen oder verzweigten, gesättigten oder ungesättigten aliphatischen Rest mit bis zu 6 Kohlenstoffatomen, der gegebenenfalls die folgenden Substituenten tragen kann: Halogenatome, Cyan-, Alkoxy- und Alkylthio-Gruppen, wobei die beiden letzteren bis zu 4 Kohlenstoffatome besitzen können, ferner Phenyl-, Phenoxy- und heterocyclische Gruppen, wobei die letzteren 5 oder 6 Ringglieder enthalten können, gesättigt oder ungesättigt sein und Sauerstoff- oder Schwefel- und/oder Stickstoffatome als Heteroatome enthalten, zu nennen sind z.B. die Furyl-Gruppe und die Pyranyl-Gruppe (Abkömmlinge des Pyrans) und deren hydrierte und teilhydrierte Derivate, schliesslich ist als Substituent noch die Amino-Gruppe zu nennen, wobei die Wasserstoffatome derselben durch Alkyl mit bis zu 4 Kohlenstoffatomen oder durch Phenyl substituiert sein können. Weiterhin steht R noch vorzugsweise für Cycloalkyl-Gruppen mit 5 oder 6 Kohlenstoffatomen und schliesslich noch für substituiertes Phenyl, wobei als Substituenten, die gleich oder verschieden sein können, vorzugsweise zu nennen sind: Alkyl, Alkenyl und Alkinyl, sowie Alkoxy und Alkylthio mit jeweils bis zu 3 Kohlenstoffatomen, ferner Phenyl und Phenoxy, sodann Halogen, Nitro-, Cyano-, Halogenmethyl-, wie z.B. Trifluormethyl-, Halogenäthyl-, Trifluormethoxy-, Trifluormethylthio-, Thiocyanato-, Carboxy- und Alkoxycarbonyl-Gruppen mit bis zu 4 C-Atomen im Alkylteil, und schliesslich noch die Acetylamino-Gruppe.

Die Amine der Formel (III) sind bekannte Verbindungen, die in allgemein bekannter Weise erhältlich und in organisch-chemischen Laboratorien üblich sind. So wird z.B. das [Tetrahydropyranyl-(2)]-methylamin, auch als 2-Aminomethyltetrahydropyran bezeichnet, dadurch erhalten, dass man 2-Cyano-tetrahydropyran hydriert, z.B. mit Lithium-alanat oder mit Wasserstoff unter Druck (vgl. Beilstein 18, E 3/4, Seite 7045). Das 2-Aminomethyl-3,4-dihydropyran hingegen kann dargestellt werden, indem man 2-Formyl-3,4-dihydropyran unter Druck in Gegenwart von Ammoniak hydriert.

Selbstverständlich können die Amine auch in Form ihrer Säureadditionsprodukte Verwendung finden, so z.B. in Form ihrer Hydrochloride, Hydrobromide, oder in Form von Addukten mit organischen Säuren, als Beispiel sei hier die Fumarsäure genannt.

Als Verdünnungsmittel können für die erfindungsgemässe Reaktion alle üblichen polaren Lösungsmittel Verwendung finden. Vorzugsweise verwendet man Wasser, ferner Alkohole, wie z.B. Methanol, Äthanol oder Propanol, ferner Ketone, wie Aceton. Weiterhin können auch Gemische verschiedener Lösungsmittel Verwendung finden, so z.B. Mischungen bestehend aus Wasser und einem mit Wasser mischbaren Lösungsmittel.

Als Hilfsstoffe können übliche Säurebindungsmittel (Basen) verwendet werden. Hierzu gehören vorzugsweise anorganische Säurebinder, wie Alkalihydroxide und Alkalicarbonate, als Beispiele seien hier Natriumhydroxid und Kaliumcarbonat genannt. Beim Arbeiten in organischen Lösungsmitteln werden vorzugsweise tertiäre Amine verwendet, wie z.B. Triäthylamin oder Pyridin.

Vorzugsweise kann auch das als Ausgangsprodukt zu verwendende Amin der Formel (III) im Überschuss eingesetzt werden und somit gleichzeitig als Reaktionspartner und als Säurebindungsmittel fungieren.

Die Reaktionstemperaturen können in einem grösseren Bereich variiert werden. Im allgemeinen arbeitet man zwischen –5 und +150 °C, vorzugsweise zwischen +10 und 50 °C.

Bei der Durchführung des erfindungsgemässen Verfahrens setzt man auf ein Mol Dichlormaleinsäuredihalogenid der Formel IIa bzw. IIb mindestens 2 Mole des Amins der Formel (III) und mindestens 2 Mole eines Säurebindemittels ein, wobei, wie schon erwähnt, anstelle eines separaten Säurebindemittels auch das Amin der Formel (III) im Überschuss eingesetzt werden kann; im letzteren Falle werden dann mindestens 4 Mole Amin pro Mol Dichlormaleinsäuredihalogenid benötigt. Die Aufarbeitung erfolgt in gewohnter Weise, z.B. dergestalt, dass man das aus Wasser ausgefallene Endprodukt absaugt und trocknet.

Die erfindungsgemässen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemässen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger.

So zeigen sie eine besonders gute Wirksamkeit gegen den Erreger der Kraut- und Braunfäule der Tomate (Phytophthora infestans) und den eine Reiskrankheit verursachenden Pilz Pyricularia oryzae.

Die Wirkstoffe sind im Getreidebau gut wirksam gegen den Getreiderost (Puccinia recondita), den Weizensteinbrand (Tilletia caries) sowie die Streifenkrankheit der Gerste (Helminthosporium gramineum).

Als Pflanzenschutzmittel können die erfindungsgemässen Wirkstoffe zur Saatgutbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmne-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnusschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummi arabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Spritzen, Sprühen, Stäuben, Streuen, Trockenbeizen, Feuchtbeizen, Schlämmbeizen oder Inkrustieren.

Bei der Verwendung als Blattfungizide und Blattbakterizide können die Wirkstoffkonzentrationen in den Anwendungsformen in einem grösseren Bereich variiert werden. Sie liegen im allgemeinen zwischen 0,5 und 0,0005 Gew.-%, vorzugsweise zwischen 0,2 und 0,001%.

Bei der Saatgutbehandlung werden im allgemeinen Wirkstoffmengen von 0,01 bis 50 g je Kilogramm Saatgut, vorzugsweise 0,5 bis 5 g, benötigt.

Zur Behandlung sind Wirkstoffmengen von 1 bis 1000 g je cbm Boden, vorzugsweise von 10 bis 200 g, erforderlich.

Neben der fungiziden Wirkung zeigen die erfindungsgemässen Verbindungen in den entsprechenden Aufwandmengen und -konzentrationen auch eine Wirkung als Pflanzenwachstumsregulatoren.

Einige Verwendungsmöglichkeiten seien anhand der nachfolgenden Beispiele aufgezeigt:

Beispiel A:
Myzelwachstums-Test
Verwendeter Nährboden:

| | |
|---|---|
| 20 | Gewichtsteile Agar-Agar |
| 200 | Gewichtsteile Kartoffeldekokt |
| 4 | Gewichtsteile Malz |
| 15 | Gewichtsteile Dextrose |
| 5 | Gewichtsteile Pepton |
| 2 | Gewichtsteile Dinatriumhydrogenphosphat |
| 0,3 | Gewichtsteile Dicalciumnitrat |

Verhältnis von Lösungsmittelgemisch zu Nährboden:

| | |
|---|---|
| 2 | Gewichtsteile Lösungsmittelgemisch |
| 100 | Gewichtsteile Agarnährboden |

Zusammensetzung Lösungsmittelgemisch

| | |
|---|---|
| 0,19 | Gewichtsteile Dimethylformamid |
| 0,01 | Gewichtsteile Emulgator (Alkyl-aryl-polylglykoläther) |
| 1,80 | Gewichtsteile Wasser |
| 2 | Gewichtsteile Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42 °C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den unten angegebenen Pilzarten beimpft und bei etwa 21 °C inkubiert.

Die Auswertung erfolgt je nach der Wachstumsgeschwindigkeit der Pilze nach 4–10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

| | |
|---|---|
| 1 | kein Pilzwachstum |
| bis 3 | sehr starke Hemmung des Wachstums |
| bis 5 | mittelstarke Hemmung des Wachstums |
| bis 7 | schwache Hemmung des Wachstums |
| 9 | Wachstum gleich der unbehandelten Kontrolle |

Die Versuchsauswertung ergab, dass gegen die Pilzarten Sclerotinia sclerotiorum, Fusarium nivale, Colletotrichum coffeanum, Rhizoctonia solani, Verticillium alboatrum, Pyricularia oryzae, Helminthosporium gramineum, Mycosphaerella musicola, Phytophthora cactorum und Pellicularia saskii z.B. die Verbindungen gemäss Herstellungsbeispielen 1, 6, 7, 15, 14, 2, 3, 8 und 11 eine den beim Stand der Technik bekannten Verbindungen überlegene Wirkung besitzen.

Beispiel B:
Pyricularia-Test / flüssige Wirkstoffzubereitung

| | | |
|---|---|---|
| Lösungsmittel: | 11,75 | Gewichtsteile Aceton |
| Dispergiermittel: | 0,75 | Gewichtsteile Alkyl-aryl-polyglykoläther |
| Wasser: | 987,50 | Gewichtsteile |
| andere Zusätze: | – | Gewichtsteile |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man etwa 14 Tage alte Reispflanzen bis zur Tropfnässe. Die Pflanzen verbleiben bis zum Abtrocknen in einem Gewächshaus bei Temperaturen von 22 bis 24 °C und einer relativen Luftfeuchtigkeit von etwa 70%. Danach werden sie mit einer wässrigen Suspension von 100 000 bis 200 000 Sporen/ml von Pyricularia oryzae inokuliert und in einem Raum bei 24 bis 26 °C und 100% relativer Luftfeuchtigkeit aufgestellt.

5 Tage nach der Inokulation wird der Befall bei allen zur Zeit der Inokulation vorhandenen Blättern in Prozent der unbehandelten, aber ebenfalls inokulierten Kontrollpflanzen bestimmt. Die Auswertung erfolgt in Wertzahlen von 1–9. 1 bedeutet 100%ige Wirkung, 3 = gute Wirkung, 5 = mässige Wirkung und 9 = keine Wirkung.

Bei der Auswertung ergab sich, dass z.B. die Verbindungen gemäss Herstellungsbeispielen 1, 6, 7, 15, 14, 18, 2, 3, 8 und 11 eine den beim Stand der Technik genannten Verbindungen überlegene Wirkung besitzen.

Beispiel C:
Phytophthora-Test (Tomaten)/Protektiv

| | |
|---|---|
| Lösungsmittel: | 4,7 Gewichtsteile Aceton |
| Emulgator: | 0,3 Gewichtsteile Alkylaryl-polyglykoläther |
| Wasser: | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70% im Gewächshaus. Anschliessend werden die Tomatenpflanzen mit einer wässrigen Sporensuspension von Phytophthora infestans inokuliert. Die Pflanzen werden in eine Feuchtkammer mit einer 100%igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, dass die Pflanzen vollständig befallen sind.

Die Versuchsauswertung ergab, dass z.B. die Verbindungen gemäss Herstellungsbeispielen 6, 15, 2, 10, 8, 16, 17 und 11 eine den beim Stande der Technik genannten Verbindungen überlegene Wirkung besitzen.

Herstellungsbeispiele

Beispiel 1

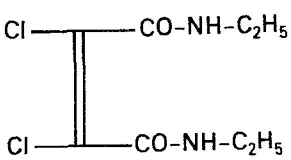

10,0 g (45,1 mMol) Tetrachlor-5-furan-2-on («Dichlormaleinsäuredichlorid») werden unter starkem Rühren bei Raumtemperatur langsam in 40 ml einer 25%igen Äthylamin-Lösung eingetragen. Das ausfallende Produkt wird abgesaugt und getrocknet. Man erhält 8,7 g Dichlormaleinsäure-bis-diäthylamid vom Fp. 154-155°C. Die Ausbeute beträgt 81% der Theorie.

Beispiel 2

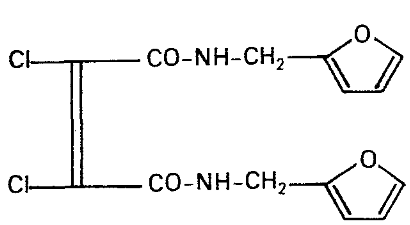

22,2 g (0,1 Mol) Dichlormaleinsäuredichlorid werden unter starkem Rühren bei Raumtemperatur langsam in eine Mischung von 20,2 g (0,2 Mol) Triäthylamin und 19,4 g (0,2 Mol) Furfurylamin in 200 ml Aceton eingetragen. Die Reaktionsmischung wird auf 1000 ml Wasser gegeben, das ausfallende Produkt abgesaugt und getrocknet. Man erhält 29,0 g Dichlormaleinsäure-bis-furfurylamid vom Fp. 149°C. Die Ausbeute beträgt 85% der Theorie.

Beispiel 3

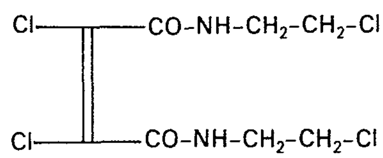

40,1 g (0,4 Mol) 2-Chloräthylamin-hydrochlorid, in 150 ml Wasser gelöst, werden mit 160 g 10%iger Natronlauge versetzt und anschliessend wird bei Raumtemperatur langsam 22,2 g (0,1 Mol) Dichlormaleinsäuredichlorid eingetragen. Das ausfallende Produkt wird abgesaugt und getrocknet. Man erhält 19,7 g Dichlormaleinsäure-bis-(2-chloräthyl)-amid vom Fp. 107-110°C, das sind 64% der Theorie.

Analog den obigen Beispielen werden die nachfolgenden Verbindungen der allgemeinen Formel

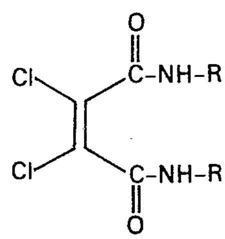 (I)

hergestellt.

| Beispiel Nr. | R | Fp. (°C) |
|---|---|---|
| 4 | $-CH_3$ | 162-163 |
| 5 | $-C_3H_7(n)$ | 135-137 |
| 6 | $-C_4H_9(n)$ | 130-132 |
| 7 | $-C_4H_9(i)$ | 166-167 |
| 8 | $-CH_2-CH=CH_2$ | 127 |
| 9 | $-CH_2CH_2CH_2OCH_3$ | 83- 85 |
| 10 | 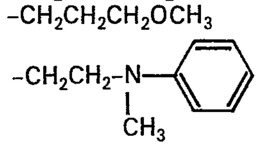 | 113-116 |
| 11 | 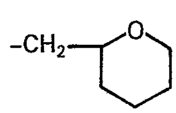 | 139-140 |
| 12 | 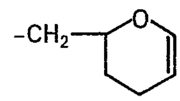 | 133-134 |
| 13 | 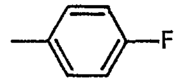 | 187-190 |

| Beispiel Nr. | R | Fp. (°C) |
|---|---|---|
| 14 | 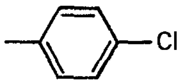 | 185 |
| 15 | 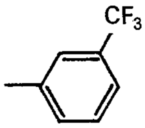 | 147–149 |
| 16 | 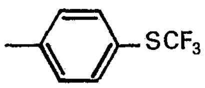 | 138–140 |
| 17 | 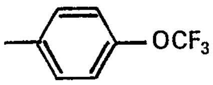 | 146 |
| 18 | 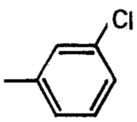 | 178 |

In entsprechender Weise können auch die folgenden Verbindungen der Formel (I) hergestellt werden, in denen R die nachfolgend genannte Bedeutung besitzt:

| R |
|---|
| $-CH_2-CH=CHCH_3$ |
| $-CH_2-C(CH_3)=CH_2$ |
| $-CH_2-C{\equiv}CH$ |

**Patentansprüche**

1. Dichlormaleinsäurediamid-Derivate mit der Formel

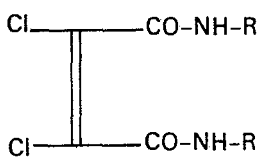 (I)

in welcher
R für einen gegebenenfalls substituierten aliphatischen Rest mit bis zu 8 Kohlenstoffatomen, für einen Cycloalkylrest mit 3 bis 6 Kohlenstoffatomen oder für einen substituierten Phenylrest steht.

2. Verfahren zur Herstellung von Dichlormaleinsäurediamid-Derivaten der Formel I in Anspruch 1, bei dem man Halogenide der Dichlormaleinsäure mit der Formel

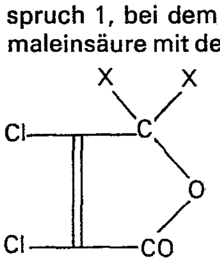 bzw. 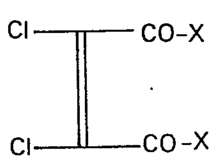

(IIa) (IIb)

in welcher
X für Halogen steht,
mit einem Amin der Formel

$H_2N-R$ (III)

in welcher
R die oben angegebene Bedeutung besitzt,
in einem Verdünnungsmittel in Gegenwart eines Säurebindemittels umsetzt.

3. Verfahren nach Anspruch 2, dadurch gekennzeichnet, dass man die Umsetzung im Temperaturbereich zwischen -5 und +150°C durchführt.

4. Verfahren nach Ansprüchen 2 und 3, dadurch gekennzeichnet, dass man die Umsetzung in der Weise vornimmt, dass man das zu acylierende Amin im Reaktionsgefäss vorlegt und das Dichlormaleinsäurehalogenid anschliessend zugibt.

5. Fungizide Mittel, dadurch gekennzeichnet, dass die Mittel einen Gehalt an mindestens einem Dichlormaleinsäurediamid-Derivat gemäss Anspruch 1 aufweisen.

6. Verwendung von Dichlormaleinsäurediamid-Derivaten gemäss Anspruch 1 zur Bekämpfung von Pilzen.

7. Verfahren zur Herstellung von fungiziden Mitteln durch Vermischen einer fungiziden Verbindung mit Streckmitteln und/oder oberflächenaktiven Mitteln, dadurch gekennzeichnet, dass man als fungizide Mittel Dichlormaleinsäurediamid-Derivate gemäss Anspruch 1 verwendet.

**Patent Claims**

1. Dichloromaleic acid diamide derivatives of the formula

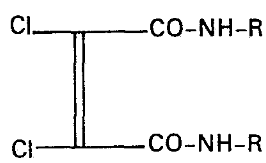 (I)

in which
R represents an optionally substituted aliphatic radical with up to 8 carbon atoms, a cycloalkyl radical with 3 to 6 carbon atoms or a substituted phenyl radcical.

2. Process for the preparation of dichloromaleic acid diamide derivatives of the formula I in Claim 1, in which dichloromaleic acid halides of the formula

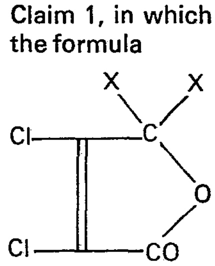 or 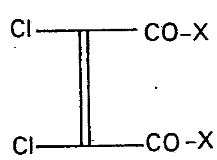

(IIa) (IIb)

in which
X represents halogen,
are reacted with an amine of the formula

$H_2N-R$ (III)

in which
R has the meaning indicated above,
in a diluent in the presence of an acid-binding agent.

3. Process according to Claim 2, characterised in that the reaction is carried out in the temperature range between -5 and +150 °C.

4. Process according to Claims 2 and 3, characterised in that the reaction is carried out by a procedure in which the amine to be acylated is initially introduced into the reaction vessel and the dichloromaleic acid halide is then added.

5. Fungicidal agents, characterised in that the agents contain at least one dichloromaleic acid diamide derivative according to Claim 1.

6. Use of dichloromaleic acid diamide derivatives according to Claim 1 for combating fungi.

7. Process for the preparation of fungicidal agents by mixing a fungicidal compound with extenders and/or surface-active agents, characterised in that dichloromaleic acid diamide derivatives according to Claim 1 are used as the fungicidal agents.

**Revendications**

1. Dérivés de diamide d'acide dichloromaléique de formule:

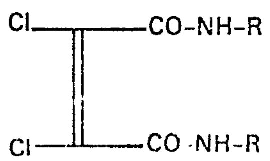

(I)

dans laquelle:
R est un reste aliphatique éventuellement substitué ayant jusqu'à 8 atomes de carbone, un reste cycloalkyle ayant 3 à 6 atomes de carbone ou un reste phényle substitué.

2. Procédé de production de dérivés de diamide d'acide dichloromaléique de formule I suivant la revendication 1, dans lequel on fait réagir des halogénures d'acide dichloromaléique de formule:

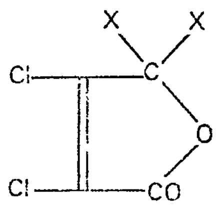

(IIa)

ou .

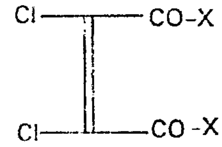

(IIb)

dans laquelle:
X est un halogène,
avec une amine de formule:

$H_2N-R$ (III)

dans laquelle:
R a la définition donnée ci-dessus
dans un diluant en présence d'un accepteur d'acide.

3. Procédé suivant la revendication 2, caractérisé en ce qu'on conduit la réaction dans la plage de températures de -5 à +150 °C.

4. Procédé suivant les revendications 2 et 3, caractérisé en ce qu'on conduit la réaction en introduisant d'abord l'amine à acyler dans le réacteur et en ajoutant ensuite l'halogénure d'acide dichloromaléique.

5. Compositions fongicides, caractérisées en ce qu'elles renferment une teneur en au moins un dérivé de diamide d'acide dichloromaléique suivant la revendication 1.

6. Utilisation de dérivés de diamide d'acide dichloromaléique suivant la revendication 1 dans la lutte contre des champignons.

7. Procédé de production de compositions fongicides par mélange d'un composé fongicide avec des diluants et/ou des agents tensio-actifs, caractérisé en ce qu'on utilise comme agents fongicides des dérivés de diamide d'acide dichloromaléique suivant la revendication 1.